# EUROPEAN PATENT SPECIFICATION

(11) **EP 0 523 182 B1**
(45) Date of publication and mention of the grant of the patent: **24.05.1995**
(21) Application number: 91908434.3
(22) Date of filing: 02.04.1991
(51) Int. Cl.: C07C 67/02, C07C 67/03, C11C 3/00, C11C 3/04, C10L 1/02

(54) **PROCESS FOR THE PRODUCTION OF FATTY ACID ALKYL ESTERS**
VERFAHREN ZUR HERSTELLUNG VON FETTSÄUREALKYLESTERN
PROCEDE DE PRODUCTION D'ESTERS ALCOYLE D'ACIDE GRAS

(30) Priority: 05.04.1990 SE 9001245
(43) Date of publication of application: 20.01.1993
(73) Proprietor: LINDQUIST, Carl-Johan, S-703 58 Örebro (SE)
(72) Inventor: LINDQUIST, Carl-Johan, S-703 58 Örebro (SE)
(74) Representative: Bjerkén, Jarl Hakan
(86) International application number: SE9100243
(87) International publication number: WO9115452

(56) References cited:
- EP-A- 0 184 740
- CH-A- 354 068

## Description

The present invention relates to a process for production of fatty acid alkyl esters according to the preamble of the appended claim 1.

Such a method is already known from EP, A2, 0 184 470 and uses an alkali metal as catalyst and the temperature is held between 50°C and 120°C.

The object of the present invention is to provide a process for production of fatty acid alkyl esters through catalytic trans-esterification of vegetable oils, which may be carried out with a minimum of energy consumption but in spite thereof efficiently, so that the process may be used to commercial production on a large scale.

This object is obtained by selecting the catalyst from the group consisting of calcium and compounds thereof, which results in the possibility to carry out the steps a)-c) of the process according to the preamble of the appended claim 1 at normal room temperature. Accordingly, the energy consumption of the process is reduced to a minimum. Nevertheless, the trans-esterification reaction has a satisfying speed.

### DETAILED DESCRIPTION OF PREFERRED EMBODIMENTS OF THE INVENTION

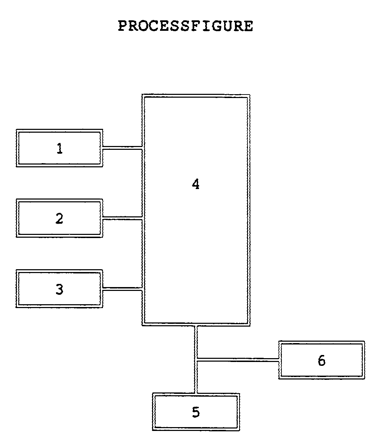

The pre-esterification process is shown in a block diagram in accordance with the figure:
1. Alcohol
2. Catalyst
3. Vegetable oil
4. Reaction vessel with mechanical stirring
5. Residual product, glycerol, alcohol and catalyst.
6. The pre-esterification product

A process for the production of simple fatty acid esters through catalytic transesterification of vegetable oil in the presence of a primary alcohol having a maximum of two carbon atoms.

The process comprises:
A. The alcohol (1), which in most of the tests started has been methanol, the quantity of which corresponded to a 10-20% stoichiometric excess, and a catalyst (2) are mixed in the reaction vessel (4) at normal atmospheric pressure and normal room temperature.
   The catalyst (2) is taken from calcium and compounds thereof.
   In the majority of tests started the catalyst (2) has been calcium, Ca.
B. The vegetable oil (3), which in all tests has been rapeseed oil, is added to the reaction vessel (4).
   The reaction starts on the condition that normal pressure and normal room temperature (15-20°C) apply.
   Reaction time increases at lower temperatures. When the reaction is complete, 80-98% of the bound glycerol has been released.
   The large variation in the glycerol obtained depends on how the stirring is carried out in the reaction vessel (4). The smaller quantity is obtained when the mixture remains unstirred.
   Two separate product phases can be identified in connection with the reaction in the reaction vessel (4), namely:
   a) The upper ester phase containing:
      85 - 97 % fatty acid alkyl esters
      3 - 15 % alcohol
      0 - 2 % glycerol
   b) The lower glycerol phase containing
      80 - 97 % glycerol
      3 - 20 % alcohol
      ...0.02 % catalyst
C. The b) phase, in a known manner, can easily be separated from the a) phase and collected in 5), while the a) phase is transferred to (6).
D. The b) phase can be returned, either in untreated condition or via distillation, in a known manner, to the reaction vessel (4) for a new start of the process.
   In this way some of the alcohol and the catalyst are reused in the process.
   The process can be carried out batchwise or continuously.

### Example 1

A mixture of 500 ml (393 g) methanol with 4 g calcium is placed in a vessel at 22°C and normal air pressure.
3300 ml (3,03 kg) rapeseed oil is added to the vessel, and the entire mixture is stirred thoroughly at 22°C and normal air pressure.
When the reaction is complete, the mixture can be separated into two phases. The upper phase contains 3,275 ml (2,880 g) methyl esters and 215 ml (170 g) methanol; while the lower phase contains 255 ml (300 g) glycerol and 89 ml (70 g) methanol.

Samples have been taken from this process.

### Example 2

A mixture of 1.6 l (1.26 kg) methanol and 8 g calcium is placed in a vessel with a mechanical stirrer. This example uses a substantially smaller amount of catalyst than the earlier example.

8.70 l (7.96 kg) rapeseed oil is added to the vessel and the entire mixture is stirred thoroughly.
Room temperature in this process varies slightly (16-20°C), while air pressure is normal.
When the reaction is complete, the mixture can be separated into two phases. The upper ester phase produces 8.93 l (7.83 kg) methyl esters and 0.63 l (0.50 kg) methanol, while the lower phase produces 0.67 l (0.80 kg) glycerol and 0.115 l (0.090 kg) methanol.

## Claims

1. A process for production of fatty acid alkyl esters through catalytic transesterification of vegetable oils in the presence of a primary alcohol having a maximum of two carbon atoms, comprising the following steps:
a) a primary alcohol having not more than two carbon atoms is mixed with a catalyst in a reaction vessel at normal air pressure,
b) a vegetable oil is then added to the reaction vessel at normal air pressure and whereupon
c) a transesterification of the vegetable oil takes place at normal air pressure and results in two phases which can be separated, said faces being an upper fatty acid ester phase and a lower glycerol phase,
**characterized** in that the steps a)-c) are carried out at normal room temperature, and that the catalyst is selected from the group consisting of calcium and compounds thereof.

2. A process according to claim 1,
**characterized** in that the lower glycerol phase obtained in step c) is reused in the process by returning it to the reaction vessel either in untreated condition or via fractionation.

3. A process according to claim 1 or 2,
**characterized** in that the mixture obtained in step b) is well stirred.

4. A process according to any of claims 1-3,
**characterized** in that the primary alcohol and the catalyst are thoroughly mixed in step a).

5. A process according to any of claims 1-4,
**characterized** in that said lower glycerol phase contains glycerol, said primary alcohol and the catalyst.

6. A process according to claim 5,
**characterized** in that the contents of the glycerol and the primary alcohol in the lower glycerol phase are 80-97% by weight and 3-20% by weight, respectively.

7. A process according to any of claims 1-6,
**characterized** in that the primary alcohol is methanol.

8. A process according to any of claims 1-7,
**characterized** in that the vegetable oil is rapeseed-oil.

9. A process according to any of claims 1-8,
**characterized** in that the upper fatty acid ester phased contains fatty acid alkyl esters and the primary alcohol.

10. A process according to claim 9,
**characterized** in that the upper fatty acid ester phase also contains glycerol.

11. A process according to claim 9 or 10,
**characterized** in that the contents of fatty acid alkyl esters, the primary alcohol and glycerol in said upper fatty acid ester phase are 85-97% by weight, 3-15% by weight and 0-2% by weight, respectively.

12. A process according to any of claims 1-11,
**characterized** in that the primary alcohol is mixed with the vegetable oil in a stoichiometric excess of 10-20% with respect to the latter.

## Patentansprüche

1. Verfahren zur Herstellung von Fettsäurealkylestern durch katalytische Umesterung von pflanzlichen Ölen unter Anwesenheit eines primären Alkohols mit maximal zwei Kohlenstoffatomen, umfassend die folgenden Schritte:
a. der primäre Alkonol mit mehr als zwei Kohlenstoffatomen wird in einem Reaktionsgefäß bei normalem Luftdruck mit einem Katalysator gemischt,
b. danach wird pflanzliches Öl bei normalem Luftdruck in das Reaktionsgefäß gegeben,
c. worauf eine Umesterung des pflanzlichen Öls bei normalem Luftdruck stattfindet, die zwei Phasen zur Folge hat, die voneinander trennbar sind und die aus einer oberen Fettsäureesterphase und einer unteren Glyzerinphase bestehen,
**dadurch gekennzeichnet**, daß die Schritte a bis c bei normaler Raumtemperatur durchgeführt werden und daß der Katalysator ausgewählt ist aus der Gruppe, die Kalzium und dessen Verbindungen enthält.

2. Verfahren nach Anspruch 1, **dadurch gekennzeichnet**, daß die im Schritt c gewonnene Glyzerzinphase in dem Verfahren wiederverwendet wird, indem sie entweder unbehandelt oder nach Fraktionierung in das Reaktionsgefäß zurückgeführt wird.

3. Verfahren nach Anspruch 1 oder 2, **dadurch gekennzeichnet**, daß die im Schritt b erzeugte Mischung gut gerührt wird.

4. Verfahren nach einem der Ansprüche 1 bis 3, **dadurch gekennzeichnet**, daß der primäre Alkohol und der Katalysator im Schritt a gründlich gemischt werden.

5. Verfahren nach einem der Ansprüche 1 bis 4, **dadurch gekennzeichnet**, daß die untere Glyzerinphase Glyzerin, den primären Alkohol und den Katalysator enthält.

6. Verfahren nach Anspruch 5, **dadurch gekennzeichnet**, daß die untere Glyzerinphase 80 bis 97 Gew.% Glyzerin und 3 bis 20 Gew.% primären Alkohol enthält.

7. Verfahren nach einem der Ansprüche 1 bis 6, **dadurch gekennzeichnet**, daß der primäre Alkohol Methanol ist.

8. Verfahren nach einem der Ansprüche 1 bis 7, **dadurch gekennzeichnet**, daß das pflanzliche Öl Rapsöl ist.

9. Verfahren nach einem der Ansprüche 1 bis 8, **dadurch gekennzeichnet**, daß die obere Fettsäureesterphase Fettsäurealkylester und den primären Alkohol enthält.

10. Verfahren nach Anspruch 9, **dadurch gekennzeichnet**, daß die obere Fettsäureesterphase auch Glyzerin enthält.

11. Verfahren nach Anspruch 9 oder 10, **dadurch gekennzeichnet**, daß die obere Fettsäureesterphase 85 bis 97 Gew.% Fettsäurealkylester, 3 bis 15 Gew.% primären Alkohol und 0 bis 2 Gew.% Glyzerin enthält.

12. Verfahren nach einem der Ansprüche 1 bis 11, **dadurch gekennzeichnet**, daß der primäre Alkohol mit dem pflanzlichen Öl in einem stöchiometrischen Überschuß von 10 bis 20 % bezüglich des Öls gemischt wird.

## Revendications

1. Procédé de production d'esters alkyliques d'acide gras par transestérification catalytique d'huiles végétales en présence d'un alcool primaire ayant au plus deux atomes de carbone, comprenant les étapes suivantes :
a) un alcool primaire ayant au plus deux atomes de carbone est mélangé avec un catalyseur dans un réacteur sous la pression normale d'air,
b) une huile végétale est ensuite ajoutée dans le réacteur sous la pression normale d'air, d'où il résulte que
c) une transestérification de l'huile végétale a lieu sous la pression normale d'air et produit deux phases qui peuvent être séparées, les dites phases étant une phase ester d'acide gras supérieure et une phase glycérol inférieure,
caractérisé en ce que les étapes a) à c) sont effectuées à la température ambiante normale, et en ce que le catalyseur est choisi dans le groupe constitué par le calcium et ses composés.

2. Procédé selon la revendication 1, caractérisé en ce que la phase glycérol inférieure obtenue dans l'étape c) est réutilisée dans le procédé en la recyclant vers le réacteur soit à l'état non traité, soit par fractionnement.

3. Procédé selon la revendication 1 ou 2, caractérisé en ce que le mélange obtenu dans l'étape b) est bien agité.

4. Procédé selon l'une quelconque des revendications 1 à 3, caractérisé en ce que l'alcool primaire et le catalyseur sont mélangés soigneusement dans l'étape a).

5. Procédé selon l'une quelconque des revendications 1 à 4, caractérisé en ce que la dite phase glycérol inférieure contient du glycérol, le dit alcool primaire et le catalyseur.

6. Procédé selon la revendication 5, caractérisé en ce que les teneurs en glycérol et en alcool primaire dans la phase glycérol inférieure sont respectivement de 80-97 % en poids et 3-20% en poids.

7. Procédé selon l'une quelconque des revendications 1 à 6, caractérisé en ce que l'alcool primaire est le méthanol.

8. Procédé selon l'une quelconque des revendications 1 à 7, caractérisé en ce que l'huile végétale est l'huile de colza.

9. Procédé selon l'une quelconque des revendications 1 à 8, caractérisé en ce que la phase supérieure ester d'acide gras contient des esters alkyliques d'acide gras et l'alcool primaire.

10. Procédé selon la revendication 9, caractérisé en ce que la phase supérieure ester d'acide gras contient également du glycérol.

11. Procédé selon la revendication 9 ou 10, caractérisé en ce que les teneurs en esters alkyliques d'acide gras, en alcool primaire et en glycérol de la dite phase supérieure ester d'acide gras sont respectivement de 85-97 % en poids, 3-15 % en poids et 0-2 % en poids.

12. Procédé selon l'une quelconque des revendications 1 à 11, caractérisé en ce que l'alcool primaire est mélangé avec l'huile végétale avec un excès stoechiométrique de 10-20 % par rapport à cette dernière.
